(19) Europäisches Patentamt
European Patent Office
Office européen des brevets

(11) Veröffentlichungsnummer : **0 411 734 B1**

(12) # EUROPÄISCHE PATENTSCHRIFT

(45) Veröffentlichungstag der Patentschrift :
**04.05.94 Patentblatt 94/18**

(51) Int. Cl.$^5$ : **C07J 53/00,** A61K 31/56

(21) Anmeldenummer : **90250200.4**

(22) Anmeldetag : **06.08.90**

(54) **Antigestagene 11Beta-Aryl-16alpha, 17alpha-cyclohexano-estra-4,9-diene.**

(30) Priorität : **04.08.89 DD 331478**

(43) Veröffentlichungstag der Anmeldung :
**06.02.91 Patentblatt 91/06**

(45) Bekanntmachung des Hinweises auf die Patenterteilung :
**04.05.94 Patentblatt 94/18**

(84) Benannte Vertragsstaaten :
**AT BE CH DE DK ES FR GB GR IT LI LU NL SE**

(56) Entgegenhaltungen :
**WO-A-88/07051**
**GB-A- 1 427 645**

(73) Patentinhaber : **SCHERING AKTIENGESELLSCHAFT**
**D-13342 Berlin (DE)**

(72) Erfinder : **Kasch, Helmut, Dr.**
**Hermann-Duncker-Strasse 14**
**DDR-6902 Jena (DD)**
Erfinder : **Bertram, Gudrun**
**O-6551 Haidefeld 22 (DE)**
Erfinder : **Kurischko, Anatoli**
**Westendstrasse 2a**
**DDR-6900 Jena (DD)**
Erfinder : **Ponsold, Kurt, Prof. Dr.**
**Thomas-Mann-Strasse 13a**
**DDR-6900 Jena (DD)**

EP 0 411 734 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft 11β-Aryl-16α,17α-cyclohexano-estra-4,9-diene der allgemeinen Formel I

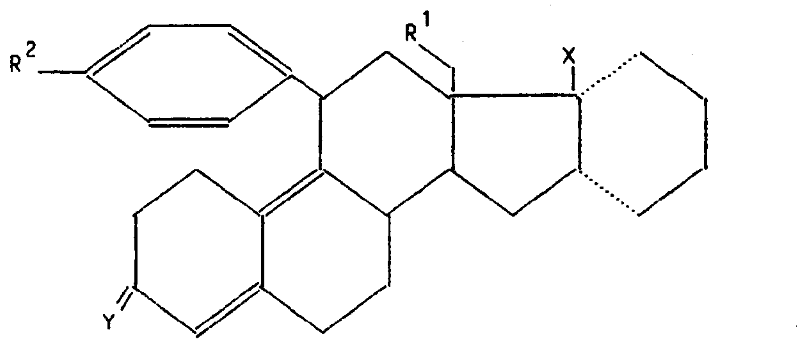

(I)

worin

- $R^1$ ein Wasserstoffatom oder eine Methylgruppe ist,
- $R^2$ = $OCH_3$, $SCH_3$, $N(CH_3)_2$, $NHCH_3$, CN, CHO, $COCH_3$, $CHOHCH_3$,
- X = CHO, $COCH_3$, $CH_2OH$, $CHOHCH_3$, $CH_2CHOAlkyl$, $CH_2CHOAlkanoyl$, $CH_2OAlkyl$, $CH_2OAlkanoyl$, COOAlkyl, wobei Alkyl und Alkanoyl eine Kohlenstoffkette mit 1 bis 7 C-Atomen bedeutet, $CH_3CH_2$, $CH_3$, COOH, CN und Y=O, NOH, $NOCH_3$ oder ein cyclisches Thioketal mit 2 oder 3 C-Ringatomen bedeutet.

Vorzugsweise bedeutet in den Verbindungen der allgemeinen Formel I $R^1$ ein Wasserstoffatom, $R^2$=$OCH_3$, $N(CH_3)_2$, CHO oder $COCH_3$ und X=CHO, $COCH_3$, $CH_2OH$ oder $CHOHCH_3$ und Y ein Sauerstoffatom oder die Hydroxyiminogruppierung NOH. Insbesondere bevorzugt sind erfindungsgemäß die folgenden Verbindungen:
16α,17α-Cyclohexano-11β-(4-methoxyphenyl)-19-nor-pregna-4,9-dien-3,20-dion,
16α,17α-Cyclohexano-11β-(4-dimethylaminophenyl)-19-nor-pregna-4,9-dien-3,20-dion,
16α,17α-Cyclohexano-20β-hydroxy-11β-(4-methoxyphenyl)-19-nor-pregna-4,9-dien-3,20-dion,
16α,17α-Cyclohexano-11β-(4-dimethylaminophenyl)-20β-hydroxy-19-nor-pregna-4,9-dien-3,20-dion,
16α,17α-Cyclohexano-17β-hydroxymethyl-11β-(4-methoxyphenyl)-estra-4,9-dien-3-on,
16α,17α-Cyclohexano-11β-(4-dimethylaminophenyl)-17β-hydroxymethyl-estra-4,9-dien-3-on,
11β-(4-Acetylphenyl)-16α,17α-cyclohexano-17β-hydroxymethyl-estra-4,9-dien-3-on,
16α,17α-Cyclohexano-11β-(4-dimethylaminophenyl)-17β-formyl-estra-4,9-dien-3-on.

Die Verbindungen der allgemeinen Formel I besitzen eine starke Affinität zum Gestagenrezeptor, ohne selbst gestagene Aktivität zu entfalten. Sie sind kompetitive Antagonisten des Progesterons (Antigestagene). In erster Linie können die erfindungsgemäßen Verbindungen zur Behandlung endokriner hormonabhängiger Tumoren, wie z. B. Mamma-Carcinom und Meningeom, aber auch zur Behandlung der Endometriose und der Dysmenorrhoe verwendet werden.

Da die Verbindungen der allgemeinen Formel I das zur Aufrechterhaltung der Schwangerschaft erforderliche Progesteron vom Rezeptor verdrängen, sind sie neben den bereits genannten Indikationen auch zur Auslösung von Aborten und zur Einleitung der Geburt geeignet.

Zur Kennzeichnung der antigestagenen Wirkung dient neben der Bestimmung der Affinität zum Progesteronrezeptor die tierexperimentell ermittelte abortive Wirkung der Substanzen.

Die abortive Wirung wurde an weiblichen graviden Ratten (positiver Spermiennachweis = 1. Graviditätstag) im Gewicht zwischen 180 und 200 g durch subkutane Gabe der in Erdnußöl suspendierten Testverbindung am 5. bis 8. Graviditätstag ermittelt. Nach Autopsie am 20. Graviditätstag wurden die Uterie untersucht. Dabei wurde die Anzahl der graviden Weibchen und die durchschnittliche Zahl der Feten pro gravides Tier festgestellt.

Der Hemmeffekt wurde wie folgt berechnet:

$$He = \left(1 - \frac{x_V \cdot n_K}{n_V \cdot x_K}\right) \cdot 100\ (\%)$$

x = Anzahl der graviden Weibchen
n = Anzahl der besamten Weibchen

V = Versuchsgruppe
K = Kontrollgruppe

| Gruppe Substanz | Gesamtdosis (mg/Tier/4d) | N | Fetilitätshemmung absol. | rel. % |
|---|---|---|---|---|
| $NMe_2$, 17β-COMe | 10 | 7 | 5 | 71 |
| $NMe_2$, 17β-$CH_2OH$ | 10 | 5 | 4 | 80 |
| Kontrolle | -- | 6 | 0 | 0 |

Die Affinität zum Progesteronrezeptor wurde durch competitive Bindung der zu testenden Substanz und eines geeigneten [$^3$H]-markierten Progestins [$^3$H]-ORG 2058 am Rezeptor untersucht.

Als biologisches Material wurde Uterus-Cytosol, frisch oder bei -20°C gefroren, erhalten von infantilen Kaninchen (Neuseeländer, Deutsche Riesen) 1 Woche nach 5-tägigem Primen mit Estradiolbenzoat (4 x 5 mg u. 1 x 10 mg) verwendet. Die Cytosol-Konzentration im Test entsprach 20 mg Gewebe/ml Inkubationsvolumen. Die Inkubationszeit betrug 20 h bei 0°C.

Nach Adsorption der freien Steroide durch Aktivkohle/Dextran und Messung des proteingebundenen [$^3$H] im Überstand, wurde $I_{50}$ [nM] ermittelt sowie die relative Bindungsaffinität RBA, bezogen auf den Standard, Progesteron, bestimmt.

$$RBA_{St.} = \left(\frac{I_{50}\ Prog.}{I_{50}\ St.}\right) \cdot 100\ [\%]$$

| Gruppe Substanz | RBA [%] |
|---|---|
| $OCH_3$, 17β-COMe | 345 |
| $NMe_2$, 17β-COMe | 470 |
| $OCH_3$, 17β-$CH_2OH$ | 170 |
| $NMe_2$, 17β-$CH_2OH$ | 220 |

Die erfindungsgemäßen Verbindungen der allgemeinen Formel I können in Form pharmazeutischer Präparate Verwendung finden. Die Herstellung der Präparate erfolgt nach an sich bekannten Methoden der Galenik durch Mischen mit organischen oder anorganischen inertem Trägermaterial, welches für die enterale, perkutane oder parenterale Applikation geeignet ist.

Die Dosierung der erfindungsgemäßen Verbindungen für die angegebenen Indikationen liegt zwischen 1 und 1000 mg täglich.

Die Verbindungen der allgemeinen Formel I werden erfindungsgemäß hergestellt, indem eine Verbindung der allgemeinen Formel V

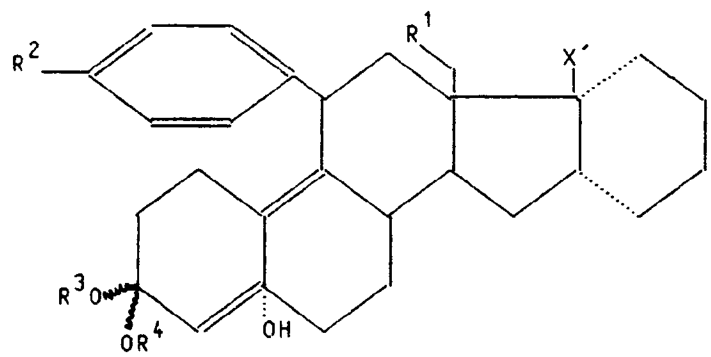

(V)

worin $R^1$ ein Wasserstoffatom oder eine Methylgruppe und $R^3$ und $R^4$ je eine Methyl- odere Ethylgruppe oder gemeinsam eine Ethylen- oder 2,2-Dialkylpropylengrupppe, insbesondere 2,2-Dimethylpropylengruppe bedeuten, sowie $R^{2'}$ und X' dieselbe Bedeutung wie $R^2$ und X in Formel I haben, wobei gegebenenfalls vorhandene Ketogruppen geschützt sind, durch Säurebehandlung in einem mit Wasser mischbaren Lösungsmittel in 11β-Aryl-16α,17α-Cyclohexano-estra-4,9-dien-3-one der allgemeinen Formel I überführt und diese anschließend gegebenenfalls durch Acylierung, Aroylierung, Oxidation, Thioketalisierung oder Oximierung zu einer anderen Verbindung der allgemeinen Formel I derivatisiert wird.

Als Säuren für die Säurebehandlung werden z.B. wäßrige Essigsäure, p-Toluolsulfonsäure oder Mineralsäuren wie Salzsäure, Schwefelsäure, Phosphorsäure oder Perchlorsäure und als Lösungsmittel wäßriges Methanol, Ethanol oder Aceton verwendet. Gegebenenfalls wird während der Säurebehandlung auf 60°C bis 70°C erwärmt.

Die weitere Derivatisierung erfolgt durch Oximierung oder Thioketalisierung in 3-Stellung und bei Vorhandensein einer 20-Hydroxygruppe gegebenenfalls durch deren Acylierung, Aroylierung oder Oxidation, wobei weitere erfindungsgemäße Verbindungen der allgemeinen Formel I gebildet werden.

20-Hydroxyethyl- oder 20-Hydroxymethyl-estra-4,9-diene der allgemeinen Formel I werden vorzugsweise in Gegenwart einer Pyridinbase mit einem Säureanhydrid oder Säurechlorid acyliert oder aroyliert.

Die Thioketalisierung wird mit Ethandithiol oder Propandithiol in Gegenwart einer Lewis-Säure, bevorzugt Bortrifluoridetherat vorgenommen.

Bei der Oxidation der 20-Hydroxyverbindungen zu den entsprechenden Ketonen, Aldehyden oder Säuren wird bevorzugt mittels Chromsäure in Aceton (Jones-Oxidation) oder aber mit Pyridiniumdichromat oder Pyridiniumchlorochromat in Methylenchlorid unter Zusatz von Natriumacetat und Molsieb gearbeitet. Bei der Herstellung von 11β-Dimethylaminophenyl-estra-4,9-dienen wird die Oxidation vorzugsweise vor Einführung des 11β-Arylrestes, nach der Ketalisierung, vorgenommen.

Die Oximierung erfolgt mit Hydroxylaminhydrochlorid oder Methoxyaminhydrochlorid in alkoholischer Lösung in Gegenwart einer Base wie $Na_2CO_3$, $K_2CO_3$, verdünnter NaOH oder KOH.

die Herstellung der erfindungsgemäß zu verwendenden Ausgangsprodukte der allgemeinen Formel V erfolgt gemäß nachstehendem Schema:

Lit. für II: Patentschrift DD 277685 A1 vom 05.12.88
(H. Kasch, Ponsold, K. u. Bertram, G; Verfahren zur Herstellung von 16α,17α-Cyclohexano-estra-4,9-dien-.3-onen)
und Patenschrift DD 251142 A1 vom 29.11.84
(K. Ponsold, Kasch, H., Kurischko, A., Stölzner, W., Kamernitzky, A., Levina, J. S., Nikitina, G., Korchow, W.; Verfahren zur Herstellung von 16α,17α-Cyclohexano-17β-acetyl-gonen-3-onen).

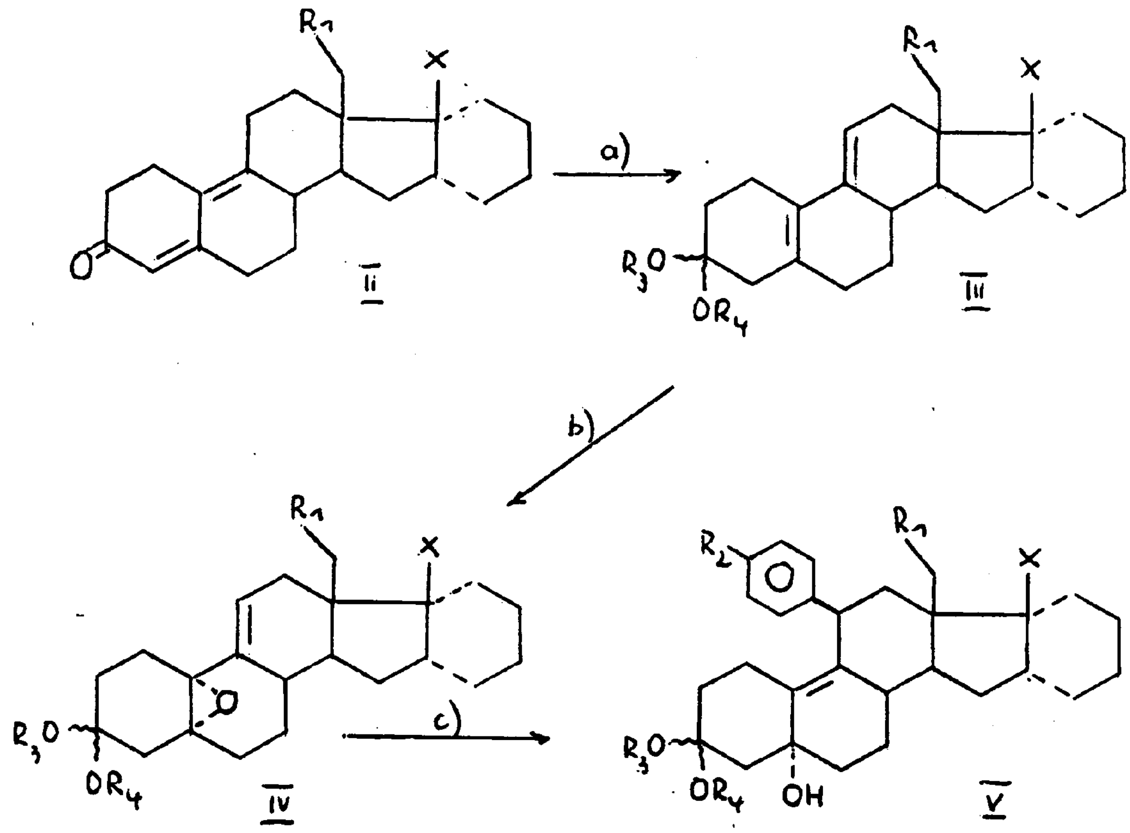


Man geht aus von den 16α,17α-Cyclohexano-estra-4,9-dienen II, die durch Ketalisierung zu III und Epoxidierung zunächst in 16α,17α-Cyclohexano-5α,10α-epoxy-3-ketale IV überführt und anschließend durch Grignardierung mit Arylmagnesiumhalogenid in Gegenwart von $Cu^{I}$-Salzen bei einer Temperatur von -30°C bis +30°C in 11β-Aryl-16α,17α-cyclohexano-5α-hydroxy-estr-9-ene V umgewandelt werden.

Die Ketalisierung wird mit einem Alkohol, bevorzugt mit Methanol, Ethanol, Ethylenglykol oder 2,3-Dimethyl-propandiol in Gegenwart katalytischer Säuremengen, beispielsweise von p-Toluolsulfonsäure, Schwefelsäure, Oxalsäure oder Pyridiniumtosylat und einem wasserentziehenden Mittel wie Ameisensäuretrimethyl- oder Ameisensäuretriethylester durchgeführt. Anstelle von Orthoameisensäureester wird vorteilhaft mit einem Wasserschleppmittel, wie Chloroform, Benzol oder Toluol am Wasserabscheider gekocht.

In einer bevorzugten Ausführungsform wird die Epoxidierung mittels Persäure vorzugsweise m-Chlorperbenzoesäure, Per-benzoesäure oder Monoperphthalsäure, in einem Zweiphasensystem bestehend aus einem organischen Lösungsmittel, wie Chloroform, Methylenchlorid, Ether, Benzol oder Toluol, und einer gesättigten wäßrigen Natriumcarbonatlösung unter kräftigem Rühren durchgeführt. Für die Grignardierung wird ein Phenylmagnesiumhalogenid verwendet, welches in p-Stellung zum Magnesium eine $OCH_3$-, $SCH_3$-, $N(CH_3)_2$-, $NHCH_3$-, CN-, $CH_3CHOH$-, $CH(OR_4)$-Gruppe enthält und worin Halogen = Brom oder Chlor bedeutet. Dabei wird in einem Ether wie Diethylether, Tetrahydrofuran oder Dioxan, gegebenenfalls unter Zusatz eines Lösungsvermittlers wie etwa Benzol oder Toluol gearbeitet. Als $Cu^{I}$-Salz kommen beispielsweise CuCN, CuJ oder CuCl infrage.

Es wurde gefunden, daß die Epoxidierung überraschenderweise mittels Persäure beim Arbeiten in einem Zweiphasensystem in Gegenwart einer wäßrigen Bicarbonatlösung gelingt und eine Zersetzung des säureempfindlichen 5α,10α-Epoxids dadurch vermieden wird.

Die nachstehenden Beispiele dienen der näheren Erläuterung der Erfindung:

## BEISPIEL 1

a) 16α,17α-Cyclohexano-3,3-ethylendioxy-19-nor-pregna-5(10),9(11)-dien-20-on

0,9 g 16α,17α-Cyclohexano-19-nor-pregna-4,9-dien-3-on werden nach Zugabe von 5 ml Glykol, 70 ml Benzol und 0,2 g p-Toluolsulfonsäure 2 Stunden am Wasserabscheider gekocht. Nach erfolgter Umsetzung wird mit Bicarbonatlösung versetzt und das Steroid mit Benzol extrahiert. Die mit Wasser neutral gewaschenen Extrakte werden eingeengt und aus Ether/n-Hexan kristallisiert, wobei 0,92 g erhalten werden.

Fp. : 124-128°C $[\alpha]_D$: 157°

b) 16α,17α-Cyclohexano-3,3-ethylendioxy-5α,10α-epoxy-19-nor-pregna-5(10),-9(11)-en-20-on

Zu einer Suspension bestehend aus 1 g 16α,17α-Cyclohexano-3,3-ethylendioxy-19-nor-pregna-5(10),9(11 )-dien-20-on in 230 ml Methylenchlorid und 20 ml einer gesättigten wäßrigen Natriumcarbonatlösung gibt man unter kräftigem Rühren 0,65 g m-Chlorperbenzoesäure. Nach erfolgter Umsetzung (15 Minuten) wird das Steroid mit Methylenchlorid extrahiert, die Extrakte eingeengt und der verbleibende Rückstand an basischem Aluminiumoxid (Greiz-Dölau) mit Toluol/Essigester (14:1 bis 9:1) flash chromatographiert. Nach Umkristallisation aus Methanol erhält man 0,4 g 5α,10α-Epoxid.

Fp. : 170-177°C

c) 16α,17α-Cyclohexano-3,3-ethylendioxy-5α-hydroxy-11β-(4-methoxyphenyl-19-nor-pregn-9-en-20-on

Von einer durch Umsetzung von 0,48 g Magnesiumspänen und 2,36 ml p-Bromanisol in 20 ml Tetrahydrofuran (THF) bei 35°C dargestellten 4-Methoxyphenylmagnesiumbromidlösung werden 5 ml entnommen und unter Argon und Kühlung auf -5°C bis -15°C mit 0,07 g CuCl versetzt. Man rührt 15 Minuten unter Beibehaltung der Kühlung und fügt dann eine Lösung von 0,3 g 16α,17α-Cyclohexano-3,3-ethylendioxy-5α,10α-epoxy-19-nor-pregn-9(11)-en-20-on in 3 ml THF tropfenweise hinzu. Anschließend wird eine Stunde bei Raumtemperatur gerührt, danach eine wäßrige Ammoniumchloridlösung zugesetzt und das Steroid mit Methylenchlorid extrahiert. Nach dem Einengen der Extrakte wird der Rückstand an basischem Aluminiumoxid (Greiz-Dölau) mit Benzol/Essigester (40:1) flash chromatographiert. Man erhält 0,297 g (78,5% d.Th. ) der 11β-Anisylverbindung.

Fp.: 152-160°C $[\alpha]_D$: 22,4°

d) 16α,17α-Cyclohexano-11β-(4-methoxyphenyl)-19-nor-pregna-4,9-dien-3,20-dion

0,2 g 16α,17α-Cyclohexano-3,3-ethylendioxy-5α-hydroxy-11β-(4-methoxyphenyl-19-nor-pregn-9-en-20-on werden in 3 ml 70%iger wäßriger Essigsäure gelöst und ca. 2 Stunden bei 60°C auf dem Wasserbad erwärmt. Gegen Ende der Reaktion beginnt das Steroid aus der Lösung zu kristallisieren. Durch Zugabe von Wasser wird das Steroid vollständig ausgefällt und nach Abtrennung an neutralem Aluminiumoxid mit Benzol/Essigester (20:1) chromatographiert. Es werden 0,146 g des 11β-Anisyl-4,9-diens erhalten, welches aus Methanol/Wasser kristallisiert werden kann.

Fp.: 190-193°C $[\alpha]_D$: 171,5°

**BEISPIEL 2**

Stufe a) und Stufe b) wird wie im Beispiel 1 hergestellt.

c) 16α,17α-Cyclohexano-11β-(4-dimethylaminophenyl)-3,3-ethylendioxy-5α-hydroxy-19-nor-pregn-9-en-20-on

Zu einer Suspension von 0,48 g Magnesiumspänen in 10 ml THF fügt man 0,5 ml Methyliodid hinzu und versetzt unter Argon sukzessive mit einer Lösung von 4,2 g p-Brom-dimethylaminobenzol in 30 ml THF, wobei die Innentemperatur 50°C nicht übersteigen sollte. Von der so erzeugten p-Dimethylaminophenylmagnesiumbromidlösung entnimmt man 15 ml und versetzt unter Kühlung (-15°C) mit 0,15 g CuCl. Man rührt etwa 15 Minuten unter Beibehaltung dieser Temperatur und fügt dann eine Lösung von 0,328 g 16α,17α-Cyclohexano-3,3-ethylendioxy-5α,10α-epoxy-19-nor-pregna-9(11),-en-20-on in 5 ml THF tropfenweise hinzu. Anschließend wird 1 Stunde bei einer Temperatur von etwa 0°C gerührt, danach eine wäßrige Ammoniumchloridlösung zugesetzt und das Steroid mit Methylenchlorid extrahiert. Nach dem Einengen der Extrakte wird der Rückstand an basischem Aluminiumoxid (Greiz-Dölau) mit Benzol/Essigester (20:1) flash chromatographiert. Man erhält 0,4 g (94,2% d.Th.) der 11β-Dimethylaminophenylverbindung, die aus Methanol umkristallisiert werden kann.

Fp.: 145-149°C $[\alpha]_D$: 31,5°

d) 16α,17α-Cyclohexano-11β-(4-dimethylaminophenyl)-19-nor-pregna-4,9-dien-3,20-dion

0,4 g (0,76 mmol) 16α,17α-Cyclohexano-11β-(4-dimethylaminophenyl)-3,3-ethylendioxy-5α-hydroxy-19-nor-pregn-9-en-20-on werden in 5 ml 70%iger wäßriger Essigsäure suspendiert und 2 Stunden bei 60°C Wasserbadtemperatur unter Rühren erwärmt. Dabei löst sich das Steroid sukzessive auf und es wird nach erfolgter Umsetzung in kaltes Wasser eingerührt, das etwas Ammoniak zur Neutralisation der Säure enthält. Das ausgefallene Produkt wird abgetrennt und an neutralem Aluminiumoxid mit Benzol/Essigester (20:1 bis 10:1) flash chromatographiert. Nach Kristallisation aus Methanol/Wasser erhält man 0,308 g (86,3%) des 4,9-Diens.

Fp.: 121 bis 126°C $[\alpha]_D$: 323,9°

**BEISPIEL 3**

a) 16α,17α-Cyclohexano-3,3-ethylendioxy-19-nor-pregna-5(10),9(11)-dien-20β-ol

3,5 g 16α,17α-Cyclohexano-19-nor-pregna-4,9-dien-20β-ol werden in 75 ml Benzol gelöst und nach Zugabe von 5 ml Glykol und 0,2 g p-Toluolsulfon säure 2 Stunden am Wasserabscheider gekocht. Nach erfolgter Umsetzung wird mit wäßriger Natriumbicarbonatlösung versetzt und das Steroid mit Benzol extrahiert. Die mit Wasser neutral gewaschenen Extrakte werden eingeengt und aus Ether/n-Hexan kristallisiert, wobei 3,3 g Ketal erhalten werden.

Fp.: 130-133°C [α]$_D$: 156°

b) 16α,17α-Cyclohexano-3,3-ethylendioxy-5α,10α-epoxy-19-nor-pregn-9(11)-en-20β-ol

Zu einer Suspension bestehend aus 2 g 16α,17α-Cyclohexano-3,3-ethylendioxy-19-nor-pregna-5(10),9(11)-dien-20β-ol in 20 ml Methylenchlorid und 20 ml einer gesättigten wäßrigen Natriumbicarbonatlösung gibt man unter kräftigem Rühren 1,44 g m-Chlorperbenzoesäure. Nach erfolgter Umsetzung (15 Minuten) wird das Steroid mit Methylenchlorid extrahiert, die Extrakte eingeengt und der verbleibende Rückstand an basischem Aluminiumoxid (Greiz-Dölau) mit Toluol/Essigester (10:1) flash chromatographiert. Man erhält 0,9 g 5α,10α-Epoxid in Form eines Öles.

c) 16α,17α-Cyclohexano-3,3-ethylendioxy-11β-(4-methoxyphenyl)-19-nor-pregn-9-en-5α,20β-diol

Von einer durch Umsetzung von 0,48 g Magnesiumspänen und 2,36 ml p-Bromanisol in 20 ml THF bei 35°C dargestellten 4-Methoxyphenylmagnesiumbromidlösung werden 5 ml entnommen und unter Argon und Kühlung auf -5°C bis -15°C mit 0,07 g CuCl versetzt. Man rührt 15 Minuten unter Beibehaltung der Kühlung und fügt dann eine Lösung von 0,765 g 16α,17α-Cyclohexano-3,3-ethylendioxy-5α,10α-epoxy-19-nor-pregn-9(11)-en-20β-ol in 5 ml THF tropfenweise hinzu. Anschließend wird 30 Minuten bei Raumtemperatur gerührt, danach eine wäßrige Ammoniumchloridlösung zugesetzt und das Steroid mit Methylenchlorid extrahiert. Nach dem Einengen der Extrakte wird der Rückstand an basischem Aluminiumoxid (Greiz-Dölau) mit Benzol/Essigester (4:1) flash chromatographiert. Nach Umkristallisation aus Ether/n-Hexan erhält man 0,7 g (72,6% d.Th.) der 11β-Anisylverbindung.

Fp.: 187-191°C [α]$_D$: opt.inakt.

d) 16α,17α-Cyclohexano-20β-hydroxy-11β-(4-methoxyphenyl)-19-nor-pregna-4,9-dien-3-on

Herstellung analog Beispiel 1 d).

Fp.: 111-116°C [α]$_D$: 91,6°

**BEISPIEL 4**

Die Herstellung der Stufen a) und b) erfolgt analog Beispiel 3

c) 16α,17α-Cyclohexano-11β-(4-dimethylaminophenyl)-3,3-ethylendioxy-19-nor-pregn-9-en-5α,20β-diol

Zu einer Suspension von 0,48 g Magnesiumspänen in 10 ml THF fügt man 0,05 ml Methyliodid hinzu und versetzt unter Argon sukzessive mit einer Lösung von 4,2 g p-Brom-dimethylaminobenzol in 30 ml THF, wobei die Innentenperatur 50°C nicht übersteigen sollte. Von der so erzeugten p-Dimethylaminophenylmagnesiumbromidlösung entnimmt man 20 ml und versetzt unter Kühlung (-15°C) mit 0,1 g CuCl. Man rührt etwa 15 Minuten unter Beibehaltung dieser Temperatur und fügt dann eine Lösung von 0,72 g 16α,17α-Cyclohexano-3,3-ethylendioxy-5α,10α-epoxy-19-nor-pregn-9(11)-en-20β-ol in 7 ml THF tropfenweise hinzu. Anschließend wird 3 Stunden bei einer Temperatur von etwa 0°C gerührt, danach eine wäßrige Ammoniumchloridlösung zugesetzt und das Steroid mit Methylenchlorid extrahiert. Nach dem Einengen der Extrakte wird der Rückstand an basischem Aluminiumoxid (Greiz-Dölau) mit Benzol/Essigester (4:1) flash chromatographiert. Das Produkt kann aus Methanol umkristallisiert werden.

Fp.: 187-194°C [α]$_D$: 5°

d) 16α,17α-Cyclohexano-11β-(4-dimethylaminophenyl)-20β-hydroxy-19-nor-pregna-4,9-dien-3-on

0,6 g 16α,17α-Cyclohexano-11β-(dimethylamino)-3,3-ethylendioxy-19-nor-pregn-9-en-5α,20β-diol werden entsprechend Beispiel 1 d) zur Umsetzung gebracht und aufgearbeitet. Nach Kristallisation aus Methanol/Wasser werden 0.47 g (89,3%) des 4,9 Diens erhalten.

Fp.: 128-134°C [α]$_D$: 196,8°

**BEISPIEL 5**

Die Herstellung der Stufen a) und b) erfolgt analog Beispiel 3.

c) 16α,17α-Cyclohexano-3,3-ethylendioxy-11β-(4-(2'-methyl-1',3'-dioxolan-2'yl)-phenyl-19-nor-pregn-9-en-5α,20β-diol

Zu einer Suspension von 0,48 g Magnesiumspänen in 13 ml THF fügt man 0,05 Methyliodid hinzu und versetzt unter Argon sukzessive mit einer Lösung von 4,9 g p-Brom-(2'-methyl-1',3'-dioxolan-2'-yl)-benzol in 27 ml THF, wobei die Innentemperatur 45°C nicht übersteigen sollte. Nach Auflösung des Magnesiums entnimmt man 10 ml 4-(2'-Methyl-1',3'-dioxolan-2'-yl)-phenyl-magnesiumbromid und gibt dazu unter Kühlung (-5°C bis -15°C) 0,1 g CuCl. Man rührt 15 Minuten unter Beibehaltung dieser Temperatur und fügt dann eine Lösung von 0,355 g 16α,17α-Cyclohexano-3,3-ethylendioxy-5α,10α-epoxy-19-nor-pregn-9(11)-en-20β-ol in 3 ml THF tropfenweise hinzu. Anschließend wird 1 Stunde gerührt, wobei die Reaktionslösung allmählich auf Raumtemperatur gebracht wird. Nach erfolgter Umsetzung wird eine wäßrige Ammoniumchloridlösung zugesetzt und das Steroid mit Methylenchlorid extrahiert. Nach dem Einengen der Extrakte wird der verbleibende Rückstand an basischem Aluminiumoxid (Greiz-Dölau) mit Benzol/Essigester (20:1) flash chromatographiert. Man erhält 0,31 g (63% d. Th.) der 11β-Acetophenylketalverbindung, die aus Methanol umkristallisiert werden kann.
Fp.: 194-202°C [α]$_D$: opt. inakt.

**BEISPIEL 6**

a) 16α,17α-Cyclohexano-3,3-ethylendioxy-17β-hydroxymethyl-estra-5(10),9(11) dien

5,9 g 16α,17α-Cyclohexano-3,3-ethylendioxy-17β-hydroxymethyl-estra-4,9-dien-3-on werden in 100 ml Benzol und 5 ml Glykol gelöst und nach Zugabe von 0,2 g p-Toluolsulfonsäure 2 Stunden am Wasserabscheider gekocht. Nach erfolgter Umsetzung wird mit wäßriger Natriumbicarbonatlösung versetzt und das Steroid mit Benzol extrahiert. Die mit Wasser neutral gewaschenen Extrakte werden eingeengt und aus Ether/n-Hexan kristallisiert, wobei 5,5 g Ketal erhalten werden.

b) 16α,17α-Cyclohexano-3,3-ethylendioxy-17β-hydroxymethyl-5α,10α-epoxy-estr-9(11)-en

Die Herstellung erfolgt analog Beispiel 1 Stufe b)

Fp.: Aceton/n-Hexan: 171-175°C

c) 16α,17α-Cyclohexano-3,3-ethylendioxy-17β-hydroxymethyl-11β-(4-methoxyphenyl)-estr-9-en-5α-ol

Von einer durch Umsetzung von 0,48 g Magnesiumspänen und 2,36 ml p-Bromanisol in 20 ml THF bei 35°C dargestellten 4-Methoxyphenylmagnesiumbromidlösung werden 4 ml entnommen und unter Argon und Kühlung auf -5°C bis -15°C mit 0,05 g CuCl versetzt. Man rührt 15 Minuten unter Beibehaltung der Kühlung und fügt dann eine Lösung von 0,5 g 16α,17α-Cyclohexano-3,3-ethylendioxy-17β-hydroxymethyl-5α,10α-epoxy-estr-9(11)-en in 3 ml THF tropfenweise hinzu. Anschließend wird eine Stunde bei Raumtemperatur gerührt, danach eine wäßrige Ammoniumchloridlösung zugesetzt und das Steroid mit Methylenchlorid extrahiert. Mach dem Einengen der Extrakte wird der Rückstand an basischem Aluminiumoxid (Greiz-Döllau) mit Benzol/Essigester (4:1) flash chromatographiert. Nach Umkristallisation aus Methanol erhält man 0,5 g (78,7% d.Th.) der 11β-Anisylverbindung.

Fp.: 167-171°C

d) 16α,17β-Cyclohexano-17β-hydroxymethyl-11β-(4-methoxyphenyl)-estra-4,9-dien-3-on

Herstellung analog Beispiel 1 d).

Fp.: Methanol/Wasser: 114-118°C

**BEISPIEL 7**

Die Herstellung der Stufen a) und b) erfolgt analog Beispiel 6.

c) 16α,17α-Cyclohexano-11β-(4-dimethylaminophenyl)-3,3-ethylendioxy-17β-hydroxymethyl-estr-9-en-5α-ol

Zu einer Suspension von 0,48 g Magnesiumspänen in 10 ml THF fügt man 0,05 ml Methyliodid hinzu und versetzt unter Argon sukzessive mit einer Lösung von 4,2 g p-Brom-dimethylaminobenzol in 30 ml THF, wobei die Innentemperatur 50°C nicht übersteigen sollte. Von der so erzeugten p-Dimethylaminophenylmagnesiumbromidlösung entnimmt man 10 ml und versetzt unter Kühlung (-15°C) mit 0,1 g CuCl. Man rührt etwa 15 Minuten unter Beibehaltung dieser Temperatur und fügt dann eine Losung von 0,438 g 16α,17α-Cyclohexano-3,3-ethylendioxy-17β-hydroxymethyl-5α,10α-epoxy-estr-9(11)-en in 3 ml THF tropfenweise hinzu. Anschließend wird 2 Stunden bei einer Temperatur von etwa 0°C gerührt, danach eine wäßrige Ammoniumchloridlösung zugesetzt und das Steroid mit Methylenchlorid extrahiert. Nach dem Einengen der Extrakte wird der Rückstand an basischem Aluminiumoxid (Greiz-Dölau) mit Benzol/Essigester (4:1) flash chromatographiert. Man erhält 0,4 g (70% d.Th.) der 11β-Dimethylaminoverbindung, die aus Ether/n-Hexan umkristallisiert werden kann.

Fp.: 176-181°C

d) 16α,17α-Cyclohexano-11β-(4-dimethylaminophenyl)-3,3-ethylendioxy-17β-hydroxymethyl-estr-4,9-dien-3-on

0,15 g 16α,17α-Cyclohexano-11β-(4-dimethylaminophenyl)-3,3-ethylendioxy-17β-hydroxymethyl-estr-9-en-5α-ol werden in 5 ml 70%iger Essigsäure gelöst und 2 Stunden bei 60°C gerührt. Anschließend wird die Lösung mit Wasser und etwas Amoniak versetzt, worauf das Steroid in abfrittbarer Form anfällt. Das isolierte Rohprodukt wird an neutralem Aluminiumoxid mit Benzen/Essigester (4:1) flash chromatographiert. Nach Kristallisation aus Methanol/Wasser erhält man 0,1 g des 4,9-Diens.
Fp.: 130-134°C

**BEISPIEL 8**

Die Herstellung der Stufen a) und b) erfolgt analog Beispiel 6.

c) 16α,17α-Cyclohexano-3,3-ethylendioxy-17β-hydroxymethyl-11β-(4-(2'-methyl 1,3-dioxolan-2'-yl)-phenyl)-estr-9-en-5α-ol

Zu einer Suspension von 0,48 g Magnesiumspänen in 13 ml THF fügt man 0,05 ml Methyliodid hinzu und versetzt unter Argon sukzessive mit einer Lösung von 4,9 g p-Brom-(2'-methyl-1,3-dioxolan-2'-yl)-benzol in 27 ml THF, wobei die Innentemperatur 45°C nicht übersteigen sollte. Nach Auflösung des Magnesiums entnimmt man 20 ml 4-(2'-Methyl-1',3'-dioxolan-2'-yl)-phenyl-magnesiumbromid und gibt dazu unter Kühlung (-5°C bis -15°C) 0,15 g CuCl. Man rührt 15 Minuten unter Beibehaltung dieser Temperatur und fügt dann eine Lösung von 0,8 g 16α,17α-Cyclohexano-3,3-ethylendioxy-17β-hydroxymethyl-5α,10α-epoxy-estr-9(11)-en in 5 ml THF tropfenweise hinzu. Anschließend wird 3 Stunden gerührt, wobei die Reaktionslösung allmählich auf Raumtemperatur gebracht wird. Nach erfolgter Umsetzung wird eine wäßrige Ammoniumchloridlösung zugesaetzt und das Steroid mit Methylenchlorid extrahiert. Nach dem Einengen der Extrakte wird der verbleibende Rückstand an basischem Aluminiumoxid (Greiz-Dölau) mit Benzol/Essigester chromatographiert. Man erhält 0,75 g (67% d.Th.) der 11β-Acetophenylketalverbindung, die aus Methanol umkristallisiert werden kann.
Fp.: 199-204°C [α]$_D$: -10,7°

d) 11β-(4-Acetylphenyl)-16α,17α-cyclohexano-17β-hydroxymethyl-estra-4,9-dien-3-on

Die Herstellung erfolgt analog Beispiel 1 d).
Fp.: Methanol/Wasser: 132-137°C

**BEISPIEL 9**

a) 16α,17α-Cyclohexano-3,3-ethylendioxy-17β-formyl-estra-5(10),9(11)-dien

0,7 g 16α, 17α-Cyclohexano-3,3-ethylendioxy-17β-hydroxymethyl-estra-5(10),9(11)dien werden in 20 ml Methylenchlorid gelöst und mit 0,9 g Natriumacetat (wasserfrei), 0,9 g getrocknetem und gepulvertem Molsieb sowie 0,9 g Pyridiniumchlorochromat versetzt. Man rührt ca. 1 Stunde bei Raumtemperatur und filtriert die Suspension anschließend über basisches Aluminiumoxid. Nach Elution mit Methylenchlorid erhält man 0,65 g der 17β-Formylverbindung als Öl.

b) 16α,17α-Cyclohexano-3,3-ethylendioxy-17β-formyl-5α,10α-epoxy-estr-9-en

Die Herstellung erfolgt analog Beispiel 1 b).
$^3$H-NMR [ppm]: 9,6 (1H, CHO); 5,99 (1H, Vinyl); 3,01 (4H, Ketal); 0,78 (3H, 13 Me).

c) 16α,17α-Cyclohexano-3,3-ethylendioxy-17β-formyl-11β-(4-methoxyphenyl)-estra-9-en-5α-ol

Von einer durch Umsetzung von 0,49 g Magnesiumspänen und 2,36 ml Brom anisol in 20 ml THF bei 35°C dargestellten 4-Methoxyphenylmagnesiumbromidlösung werden 4 ml entnommen und unter Argon und Kühlung auf -5°C bis -15°C mit 0,5 g CuCl versetzt. Man rührt 15 Minuten unter Beibehaltung der Kühlung und fügt 0,12 g eines mit 16α,17α-Cyclohexano-3,3-ethylendioxy-17β-formyl-5α,10α-oxido-estr-9(11)-en angereicherten Produktes in 7,5 ml THF tropfenweise hinzu. Anschließend wird eine Stunde bei Raumtemperatur gerührt, danach eine wäßrige Ammoniumchloridlösung zugesetzt und das Steroid mit Methylenchlorid extrahiert. Nach dem Einengen der Extrakte wird der Rückstand an basischem Aluminiumoxid (Greiz-Dölau) flash chromatographiert. Als mobile Phase wird ein Benzol/Essigester-Gemisch (20:1) verwendet. Nach Umkristallisation aus Methanol erhält man 0,05 g (32,7% d.Th.) der 11β-Anisylverbindung.
Fp.: 170-172°C [α]$_D$: 3,3°

d) 16α,17α-Cyclohexano-3,3-ethylendioxy-17β-formyl-11β-(4-methoxyphenyl)-estr-9-an-5α-ol

0,1 g 16α,17α-Cyclohexano-3,3-ethylendioxy-17β-hydroxymethyl-11β-(4-methoxyphenyl)-estr-4-en-5α-ol werden in 10 ml Methylenchlorid gelöst. und mit 0,2 g Natriumacetat, 0,5 g getrocknetem und gepulvertem Molsieb sowie 0,3 g Pyridiniumchlorochromat versetzt. Man rührt ca. 1 Stunde bei Raumtempertur, filtriert anschließend von festen Bestandteilen ab, verdünnt mit Wasser und extrahiert das Steroid mit Methylenchlorid. Nach präparativer Schichtchromatographie an Kieselgel, als mobile Phase dient ein Benzol/Essigester-Gemisch (1:1), erhält man durch Kristallisation aus Methanol 0,88 g (80% d.Th) der

Anisylverbindung.

**BEISPIEL 10**

Die Herstellung der Stufen a) und b) erfolgt analog Beispiel 9.

c) 16α,17α-Cyclohexano-11β-(4-dimethylaminophenyl)-3,3-ethylendioxy-17β-formyl-estr-9-en-5α-ol

Zu einer Suspension von 0,48 g Magnesiumspänen in 10 ml THF fügt man 0,05 ml Methyliodid hinzu und versetzt unter Argon sukzessive mit einer Lösung von 4,2 g p-Brom-dimethylaminobenzol in 30 ml THF, wobei die Innentemperatur 50°C nicht übersteigen sollte. Von der so erzeugten p-Dimethylaminophenylmagnesiumbromidlösung entnimmt man 11,5 ml und versetzt unter Kühlung (-15°C) mit 0,065 g CuCl. Man rührt etwa 15 Minuten unter Beibehaltung dieser Temperatur und fügt dann eine Lösung von 0,41 g eines mit 16α,17α-Cyclohexano-3,3-ethylendioxy-17β-formyl-5α,10α-oxido-estr-9(11)-en angereicherten Produktes in 3 ml THF tropfenweise hinzu. Anschließend wird 3 Stunden bei einer Temperatur von etwa 0°C gerührt, danach eine wäßrige Ammoniumchloridlösung zugesetzt und das Steroid mit Methylenchlorid extrahiert. Nach dem Einengen der Extrakte wird der Rückstand an basischem Aluminiumoxid (Greiz-Dölau) flash chromatographiert. Als mobile Phase wird ein Benzol/Essigester-Gemisch (20:1) verwendet. Man erhält 0,2 g (37,5% d.Th.) der 11β-Dimethylaminoverbindung, die aus Methanol umkristallisiert werden kann.

Fp.: 165-168°C  $[\alpha]_D$: opt. inakt.

d) 16α,17α-Cyclohexano-11β-(4-dimethylaminophenyl)-17β-formyl-estra-4,9-dien-3-on

0,2 g 16α,17α-Cyclohexano-11β-(4-dimethylaminophenyl)-3,3-ethylendioxy-17β-formyl-estr-9-en-5α-ol werden entsprechend Beispiel 1 d) behandelt. Nach Chromatographie erhält man 0,15 g des 4,9-Diens, welches aus Methanol/Wasser kristallisiert werden kann.

Fp.: 113-116°C  $[\alpha]_D$: 197,8°

**BEISPIEL 11**

Die Herstellung der Stufen a) und b) erfolgt analog Beispiel 9.

c) 16α,17α-Cyclohexano-3,3-ethylendioxy-17β-formyl-11β-(4-(2'-methyl-1,3-dioxolan-2'yl)-phenyl)-19-nor-pregn-9-en-5α-ol

zu einer Suspension von 0,48 g Magnesiumspänen in 18 ml THF fügt man 0,05 ml Methyliodid hinzu und versetzt unter Argon sukzessive mit einer Lösung von 4,9 g p-Brom-(2-methyl-1,3-dioxolan-2'-yl)-benzol in 27 ml THF, wobei die Innentemperatur 45°C nicht übersteigen sollte. Nach Auflösung des Magnesiums entnimmt man 18 ml 4-(2'-Methyl-1',3'-dioxolan-2'-yl)-phenyl-magnesiumbromid und gibt dazu unter Kühlung (-5°C bis -15°C) 0,1 g CuCl. Man rührt 15 Minuten unter Beibehaltung dieser Temperatur und fügt dann eine Lösung von 0,4 g 16α,17α-Cyclohexano-3,3-ethylendioxy-17β-formyl-5α,10α-oxido-estr-9(11)-en in 3 ml THF tropfenweise hinzu. Anschließend wird 2 Stunden gerührt, wobei die Reaktionslösung allmählich auf Raumtemperatur gebracht wird. Nach erfolgter Umsetzung wird eine wäßrige Ammoniumchloridlösung zugesetzt und das Steroid mit Methylenchlorid extrahiert. Nach dem Einengen der Extrakte wird der verbleibende Rückstand an basischem Aluminiumoxid chromatographiert. Als mobile Phase wird ein Benzen/Essigester-Gemisch verwendet. Man erhält 0,39 g (64,9% d. Th.) der 11β-Acetophenylketalverbindung, die aus Methanol umkristallisiert werden kann.

Fp.: 201-206°C  $[\alpha]_D$: opt. inakt.

**Patentansprüche**

1. 11β-Aryl-16α,17α-cyclohexano-estra-4,9-diene der allgemeinen Formel I

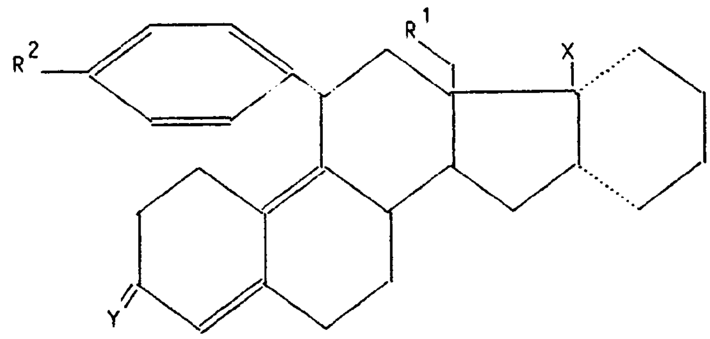

(I)

worin

$R^1$ ein Wasserstoffatom oder eine Methylgruppe ist,

$R^2$ = $OCH_3$, $SCH_3$, $N(CH_3)_2$, $NHCH_3$, CN, CHO, $COCH_3$, $CHOHCH_3$,

X = CHO, $COCH_3$, $CH_2OH$, $CHOHCH_3$, $CH_2CHOAlkyl$, $CH_2CHOAlkanoyl$, $CH_2OAlkyl$, $CH_2OAlkanoyl$, COOAlkyl, wobei Alykl und Alkanoyl eine Kohlenstoffkette mit 1 bis 7 C-Atomen bedeutet, $CH_3CH_2$, $CH_3$, COOH, CN und Y =O, NOH, $NOCH_3$ oder ein cyclisches Thioketal mit 2 oder 3 C-Ringatomen bedeutet.

**2.** Verbindungen der allgemeinen Formel I, worin $R^1$=H, $R^2$=$OCH_3$, $N(CH_3)_2$, CHO oder $COCH_3$ und X=CHO, $COCH_3$, $CH_2OH$ oder $CHOHCH_3$ und Y O oder NOH bedeuten.

**3.** Verbindungen der allgemeinen Formel I, nämlich
16α,17α-Cyclohexano-11β-(4-methoxyphenyl)-19-nor-pregna-4,9-dien-3,20-dion,
16α,17α-Cyclohexano-11β-(4-dimethylaminophenyl)-19-nor-pregna-4,9-dien-3,20-dion,
16α,17α-Cyclohexano-20β-hydroxy-11β-(4-methoxyphenyl)-19-nor-pregna-4,9-dien-3,20-dion,
16α,17α-Cyclohexano-11β-(4-dimethylaminophenyl)-20β-hydroxy-19-nor-pregna-4,9-dien-3,20-dion,
16α,17α-Cyclohexano-17β-hydroxymethyl-11β-(4-methoxyphenyl)-estra-4,9-dien-3-on,
16α,17α-Cyclohexano-11β-(4-dimethylaminophenyl)-17β-hydroxymethyl-estra-4,9-dien-3-on,
11β-(4-Acetylphenyl)-16α,17α-cyclohexano-17β-hydroxymethyl-estra-4,9-dien-3-on,
16α, 17α-Cyclohexano-11β-(4-dimethylaminophenyl)-17β-formyl-estra-4,9-dien-3-on,

**4.** Verbindungen der allgemeinen Formel V

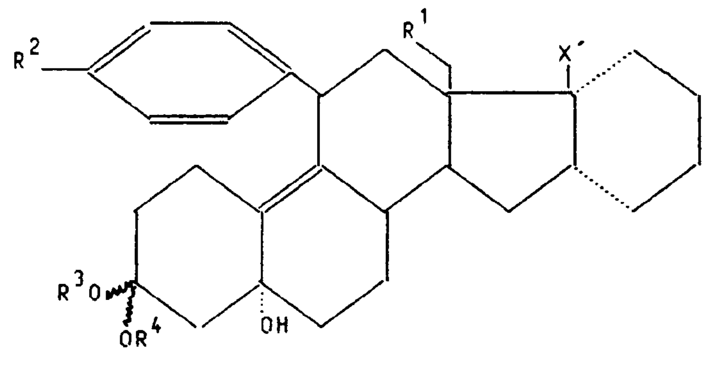

(V)

worin $R^1$ ein Wasserstoffatom oder eine Methylgruppe und $R^3$ und $R^4$ je eine Methyl- oder Ethylgruppe oder gemeinsam eine Ethylen- oder 2,2-Dialkylpropylengrupppe, insbesondere 2,2-Dimethylpropylengruppe bedeuten, sowie
$R^{2'}$ und X' dieselbe Bedeutung wie $R^2$ und X in Formel I haben, wobei gegebenenfalls vorhandene Ketogruppen geschützt sind.

**5.** Verfahren zur Herstellung von Verbindungen der allgemeinen Formel I

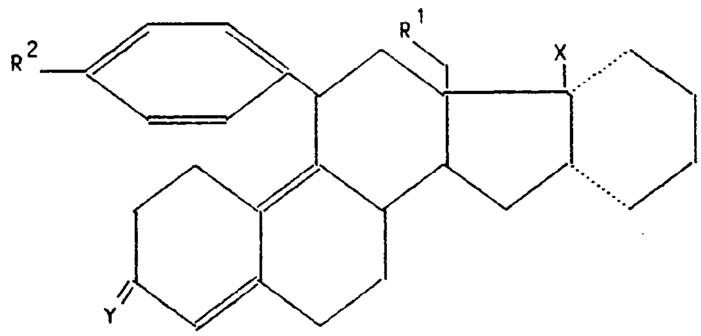

(I)

worin $R^1$, $R^2$, X und Y die in Anspruch 1 angegebene Bedeutung haben, dadurch gekennzeichnet, daß eine Verbindung der allgemeinen Formel V

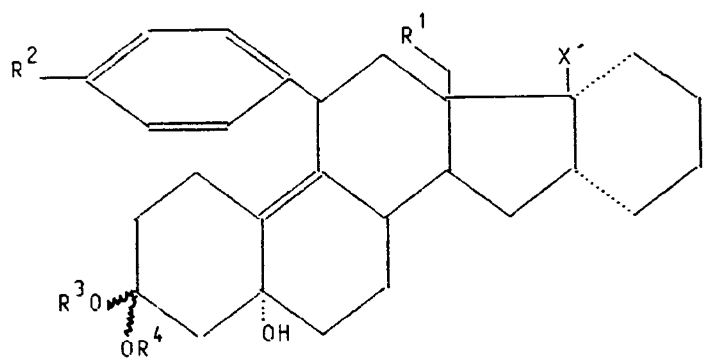

(V)

worin $R^1$ ein Wasserstoffatom oder eine Methylgruppe und $R^3$ und $R^4$ je eine Methyl- oder Ethylgruppe oder gemeinsam eine Ethylen- oder 2,2-Dialkylpropylengrupppe, insbesondere 2,2-Dimethylpropylengruppe bedeuten, sowie
$R^{2'}$ und X' dieselbe Bedeutung wie $R^2$ und X in Formel I haben, wobei gegebenenfalls vorhandene Ketogruppen geschützt sind, durch Säurebehandlung in einem mit Wasser mischbaren Lösungsmittel in 11β-Aryl-16α,17α-Cyclohexano-estra-4,9-dien-3-one der allgemeinen Formel I überführt und diese anschließend gegebenenfalls durch Acylierung, Aroylierung, Oxidation, Thioketalisierung oder Oximierung zu einer anderen Verbindung der allgemeinen Formel I derivatisiert wird.

6. Pharmazeutische Präparate, dadurch gekennzeichnet, daß sie mindestens eine Verbindung der allgemeinen Formel I sowie einen pharmazeutisch verträglichen Träger enthalten.

7. Verwendung von Verbindungen der allgemeinen Formel I zur Herstellung von Arzneimitteln.

## Claims

1. 11β-aryl-16α,17α-cyclohexano-oestra-4 ,9-dienes of the general formula I

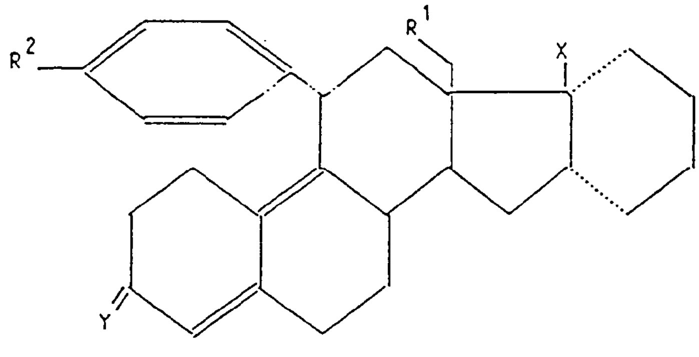

(I)

wherein

$R^1$ is a hydrogen atom or a methyl group,

$R^2$ is $OCH_3$, $SCH_3$, $N(CH_3)_2$, $NHCH_3$, CN, CHO, $COCH_3$, $CHOHCH_3$,

X is CHO, $COCH_3$, $CH_2OH$, $CHOHCH_3$, $CH_2CHO$alkyl, $CH_2CHO$-alkanoyl, $CH_2O$alkyl, $CH_2O$alkanoyl, COOalkyl, wherein alkyl and alkanoyl have a carbon chain of from 1 to 7 carbon atoms, $CH_3CH_2$, $CH_3$, COOH, CN, and

Y is O, NOH, $NOCH_3$ or a cyclic thioketal having 2 or 3 ring carbon atoms.

**2.** Compounds of the general formula I, wherein $R^1$ is H, $R^2$ is $OCH_3$, $N(CH_3)_2$, CHO or $COCH_3$ and X is CHO, $COCH_3$, $CH_2OH$ or $CHOHCH_3$, and Y is O or NOH.

**3.** Compounds of the general formula I, namely
16α,17α-cyclohexano-11β-(4-methoxyphenyl)-19-nor-pregna-4,9-diene-3,20-dione,
16α,17α-cyclohexano-11β-(4-dimethylaminophenyl)-19-nor-pregna-4,9-diene-3,20-dione,
16α,17α-cyclohexano-20β-hydroxy-11β-(4-methoxyphenyl)-19-nor-pregna-4,9-diene-3,20-dione,
16α,17α-cyclohexano-11β-(4-dimethylaminophenyl)-20β-hydroxy-19-nor-pregna-4,9-diene-3,20-dione,
16α, 17α-cyclohexano-17β-hydroxymethyl-11β-(4-methoxyphenyl)-oestra-4,9-dien-3-one,
16α,17α-cyclohexano-11β-(4-dimethylaminophenyl)-17β-hydroxymethyl-oestra-4,9-dien-3-one,
11β-(4-acetylphenyl)-16α,17α-cyclohexano-17β-hydroxymethyl-oestra-4,9-dien-3-one,
16α,17α-cyclohexano-11β-(4-dimethylaminophenyl)-17β-formyl-oestra-4,9-dien-3-one.

**4.** Compounds of the general formula V

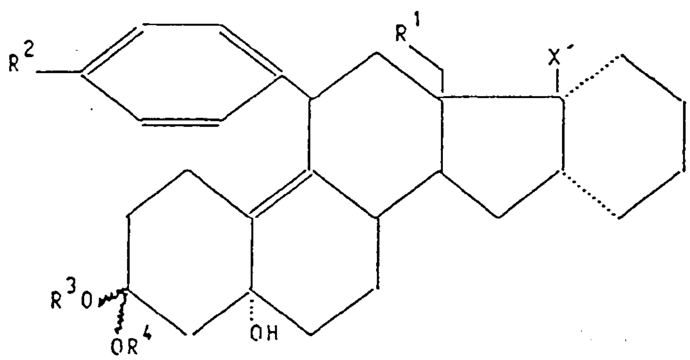

(V)

wherein $R^1$ is a hydrogen atom or a methyl group and $R^3$ and $R^4$ each represents a methyl or ethyl group or together represent an ethylene or a 2,2-dialkylpropylene group, especially a 2,2-dimethylpropylene group, and
$R^{2'}$ and X' have the same meanings as $R^2$ and X in formula I, and wherein any keto groups present are protected.

**5.** Process for the manufacture of compounds of the general formula I

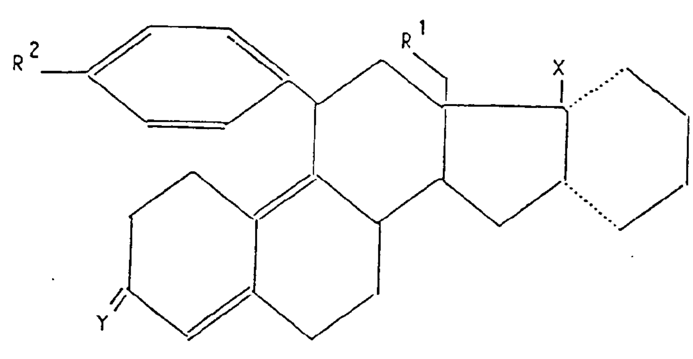

(I),

wherein $R^1$, $R^2$, X and Y have the meanings given in claim 1, characterised in that a compound of the general formula V

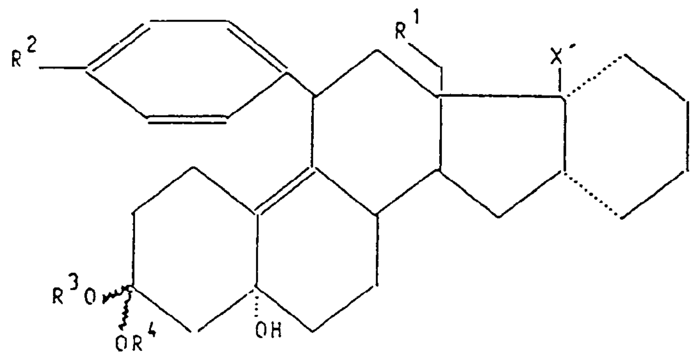

(V),

wherein $R^1$ is a hydrogen atom or a methyl group and $R^3$ and $R^4$ each represents a methyl or ethyl group or together represent an ethylene or a 2,2-dialkylpropylene group, especially a 2,2-dimethylpropylene group, and

$R^{2'}$ and X' have the same meanings as $R^2$ and X in formula I, and wherein any keto groups present are protected, is converted by acid treatment in a water-miscible solvent into an 11β-aryl-16α,17α-cyclohexano-oestra-4,9-dien-3-one of the general formula I and the latter is then optionally derivatised by acylation, aroylation, oxidation, thioketalisation or oximation to form another compound of the general formula I.

6. Pharmaceutical preparations, characterised in that they contain at least one compound of the general formula I and a pharmaceutically acceptable carrier.

7. The use of compounds of the general formula I for the manufacture of medicaments.

## Revendications

1. 11β-Aryl-16α,17α-cyclohexano-estra-4,9-diènes de formule générale I ci-dessous

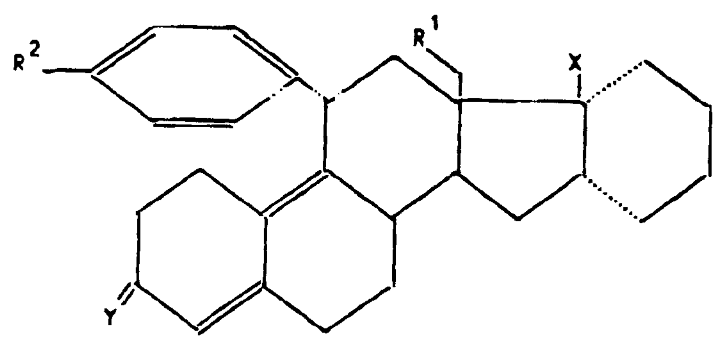

(I)

dans laquelle

- $R^1$ représente un atome d'hydrogène ou un groupe méthyle,
- $R^2$ un groupe $OCH_3$, $SCH_3$, $N(CH_3)_2$, $NHCH_3$, CN, CHO, $COCH_3$ ou $CHOHCH_3$,
- X un groupe CHO, $COCH_3$, $CH_2OH$, $CHOHCH_3$, $CH_2CHO$alkyle, $CH_2CHO$alcanoyle, $CH_2O$alkyle, $CH_2O$alcanoyle ou COOalkyle, dont les alkyles et les alcanoyles peuvent avoir de 1 à 7 atomes de carbone, ou encore un groupe $CH_3CH_2$, $CH_3$, COOH ou CN, et
- Y un atome d'oxygène, un groupe NOH ou $NOCH_3$ ou un groupe de thiocétal cyclique avec 2 ou 3 atomes de carbone dans le cycle.

2. Composés de formule I selon la revendication 1 dans lesquels $R^1$=H, $R^2$=$OCH_3$, $N(CH_3)_2$, CHO ou $COCH_3$, X=CHO, $COCH_3$, $CH_2OH$ ou $CHOHCH_3$ et Y=O ou NOH.

3. Composés de formule I selon la revendication 1, à savoir les suivants :
16α,17α-Cyclohexano-11β-(4-méthoxyphényl)-19-nor-prégna-4,9-diène-3,20-dione,

16α,17α-Cyclohexano-11β-(4-diméthylaminophényl)-19-nor-prégna-4,9-diène-3,20-dione,
16α,17α-Cyclohexano-20β-hydroxy-11β-(4-méthoxyphényl)-19-nor-prégna-4,9-diène-3,20-dione,
16α,17α-Cyclohexano-11β-(4-diméthylaminophényl)-20β-hydroxy-19-nor-prégna-4,9-diène-3,20-dione,
16α, 17α-Cyclohexano-17β-hydroxyméthyl-11β-(4-méthoxyphényl)-estra-4,9-diène-3-one,
16α, 17α-Cyclohexano-11β-(4-diméthylaminophényl)-17β-hydroxyméthyl-estra-4,9-diène-3one,
11β-(4-Acéthylphényl)-16α, 17α-cyclohexano-17β-hydroxyméthyl-estra-4,9-diène-3-one,
16α, 17α-Cyclohexano-11β-(4-diméthylaminophényl)-11β-formyl-estra-4,9-diène-3-one.

4. Composés de formule générale V

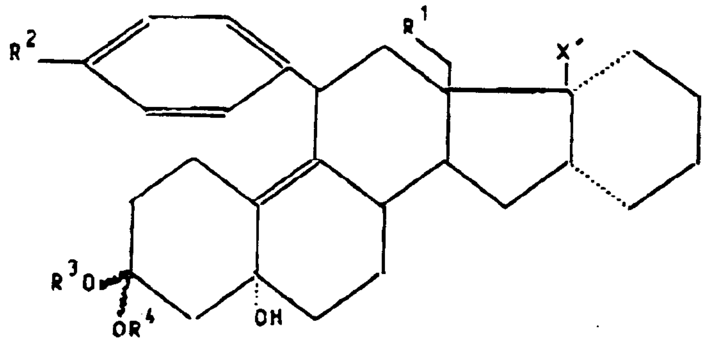

(V)

dans laquelle

| | |
|---|---|
| $R^1$ | représente un atome d'hydrogène ou groupe méthyle, |
| $R^3$ et $R^4$ | sont chacun un groupe méthyle ou éthyle ou bien forment ensemble un groupe éthylène ou 2,2-dialkylpropylène, en particulier 2,2-diméthyl-propylène, et |
| $R^{2'}$ et X' | ont les mêmes significations que $R^2$ et X dans la formule I, et des groupes céto éventuellement présents, sont protégés. |

5. Procédé de préparation des composés de formule générale I selon la revendication 1, procédé caractérisé en ce que l'on transforme un composé de formule V selon la revendication 4, par traitement avec un acide dans un solvant miscible à l'eau, en une 11β-aryl-16α, 17α-cyclohaxano-estra-4,9-diène-3-one de formule I, que le cas échéant on transforme ensuite en un autre composé de formule I par acylation, aroylation, oxydation, thiocétalisation ou oximation.

6. Préparations pharmaceutiques caractérisées en ce qu'elles contiennent un ou plusieurs composés de formule I selon la revendication 1 avec des véhicules ou excipients pharmaceutiques compatibles.

7. L'emploi des composés de formule I selon la revendication 1 pour la préparation de médicaments.